# EUROPEAN PATENT APPLICATION

(11) **EP 4 108 759 A1**
(43) Date of publication of application: **28.12.2022**
(21) Application number: 21757419.3
(22) Date of filing: 19.02.2021
(51) Int. Cl.: C12M 1/00, C12M 3/00, C12N 5/071

(54) **CELL INCUBATOR CAPABLE OF CONTROLLING CELL AGGREGATION RATE**

(30) Priority: 21.02.2020 JP 2020028120
(71) Applicant: Nissan Chemical Corporation, Tokyo 103-6119 (JP)
(72) Inventor: SUZUKI Kohei, Funabashi-shi, Chiba 274-0052 (JP); FUKASAWA, Natsuki, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2021/006244
(87) International publication number: WO 2021/167042

(57) **Abstract**

This is to provide a substrate for producing cell aggregates and a method for producing cell aggregates simply and easily, and rapidly with high mass productivity.

This is a substrate for producing cell aggregates provided with a spot(s) comprising a copolymer containing recurring units derived from monomers represented by the following formulae (I) and (II): 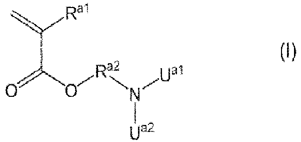 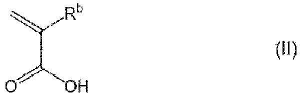 wherein U^{a1}, U^{a2}, R^{a1}, R^{a2} and R^{b} are as defined in the specification and the claims, on a substrate having an ability to inhibit adhesion of cells, wherein a completion time of forming cell aggregates after seeding cells is within 20 hours.

## Description

### TECHNICAL FIELD

The present invention relates to a substrate for producing cell aggregates capable of controlling a cell aggregation rate, which uses a polymer having a specific structure as a base coat agent for cell culture, and a method for producing the same, a method for producing cell aggregates, a method for improving a cell culturing rate at the time of producing cell aggregates, a method for controlling forming rate of aggregates at the time of producing cell aggregates, and a base coat agent of a cell culture container for promoting formation of cell aggregates.

### BACKGROUND ART

It has been reported that cell aggregates (also referred to as a spheroid or a cell mass, etc.) are three-dimensionally cultured, which are cell gathered materials in which cells are self-gathered and aggregated and a living body-like structure is constructed, so that functions of the cells can be maintained for a long term and physiological functions are improved. Therefore, expectations of cell aggregates for utilization in drug discovery research, or in cell therapy or regenerative therapy is increasing.

Accompanied with this, development of tissue engineering technology that can prepare cell aggregates simply and easily and quickly, and uniformly and a large amount is considered to be an important issue for practical application of regenerative medicine and make drug discovery test efficiency, but in the method of utilizing random aggregation phenomenon of floating cells using a conventional cell low-adhesion culture dish (for example, multi-well plates), only one spheroid is formed per one well, so that there is a problem that it is not excellent in operability and mass productivity.

In Patent Document 1, a method for producing a cell culture container using a polymer derived from poly(2-N,N-dimethylaminoethyl methacrylate) having a cationic functional group and an anionic functional group is disclosed.

### PRIOR ART DOCUMENT

### PATENT DOCUMENT

Patent Document 1: JP 2014-162865A

### SUMMARY OF THE INVENTION

### PROBLEMS TO BE SOLVED BY THE INVENTION

This is to provide a substrate for producing cell aggregates and a method for producing the same, and a method for producing cell aggregates, etc., which are for producing the above-mentioned cell aggregates simply and easily, and rapidly with high mass productivity.

### MEANS TO SOLVE THE PROBLEMS

The present invention includes the following.
[1] A substrate for producing cell aggregates provided with a spot(s) comprising a copolymer containing a recurring unit derived from a monomer represented by the following formula (I): 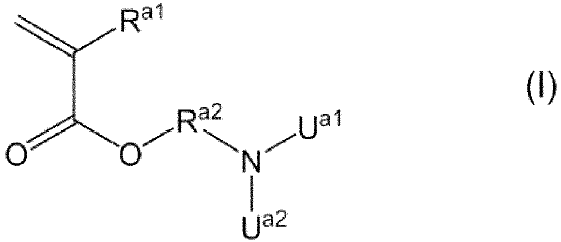 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms]
      and a recurring unit derived from a monomer represented by the following formula (II): 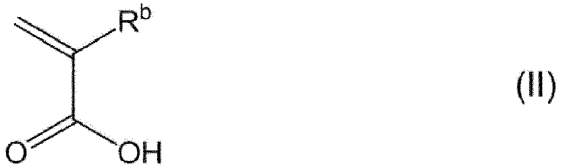 [wherein
   R^{b} represents a hydrogen atom or a linear or branched alkyl group having 1 to 5 carbon atoms]
   on a substrate having an ability to inhibit cell adhesion, wherein a completion time of forming cell aggregates after seeding cells is within 20 hours.
[2] The substrate for producing cell aggregates described in the above-mentioned [1], wherein a molar ratio of the recurring unit derived from the monomer represented by the above-mentioned formula (I) to a total of the recurring unit derived from the monomer represented by the above-mentioned formula (I) and the recurring unit derived from the monomer represented by the above-mentioned formula (II) is 99 mol% to 51 mol%.
[3] The substrate for producing cell aggregates described in the above-mentioned [1] or [2], wherein the above-mentioned copolymer further contains a structural unit derived from a monomer represented by the following formula (III): 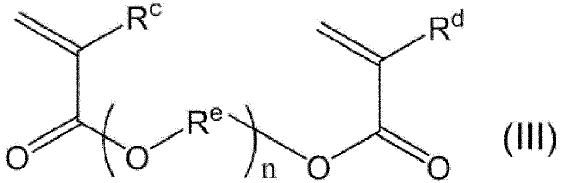 [wherein
   R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50].
[4] The substrate for producing cell aggregates described in any of the above-mentioned [1] to [3], wherein the cells are stem cells or fibroblasts.
[5] The substrate for producing cell aggregates described in any of the above-mentioned [1] to [4], wherein the substrate having an ability to inhibit cell adhesion comprises a coating film which comprises a copolymer (P) having a recurring unit containing a group represented by the following formula (a) and a recurring unit containing a group represented by the following formula (b): 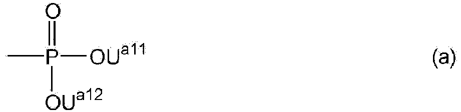 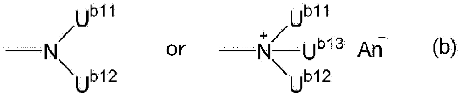 [wherein
   U^{a11}, U^{a12}, U^{b11}, U^{b12} and U^{b13} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion]
   at least a part of a surface thereof.
[6] A method for producing a substrate for producing cell aggregates, comprising steps of applying a copolymer containing a recurring unit derived from a monomer represented by the following formula (I): 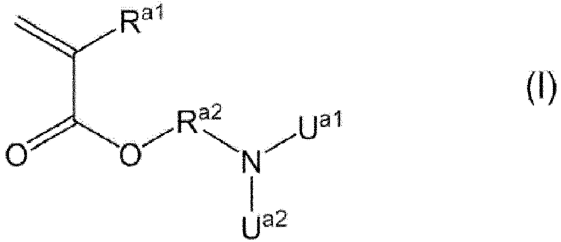 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
      and a recurring unit derived from a monomer represented by the following formula (II): 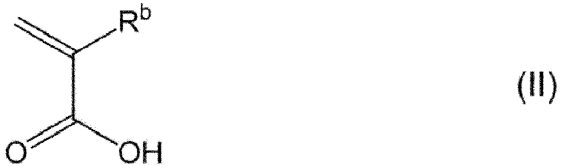 [wherein
   R^{b} represents a hydrogen atom or a linear or branched alkyl group having 1 to 5 carbon atoms]
   in a plurality of spot states to a substrate having an ability to inhibit cell adhesion, and then, drying the same, wherein a completion time of forming cell aggregates after seeding cells is within 20 hours.
[7] The method for producing a substrate for producing cell aggregates described in the above-mentioned [6], wherein the applying step is carried out by an inkjet system.
[8] A method for producing cell aggregates which comprises a step of seeding sells on a substrate for producing cell aggregates provided with a spot(s) comprising a copolymer containing a recurring unit derived from a monomer represented by the following formula (I): 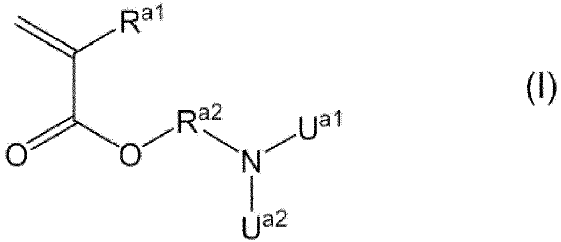 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
      and a recurring unit derived from a monomer represented by the following formula (II): 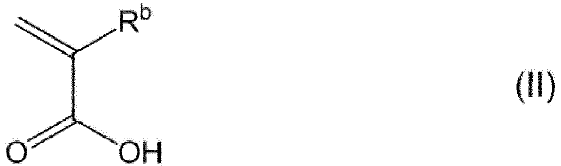 [wherein
   R^{b} represents a hydrogen atom or a linear or branched alkyl group having 1 to 5 carbon atoms]
   on the substrate having an ability to inhibit cell adhesion, and a step of culturing the cells after seeding thereof to form cell aggregates within 20 hours.
[9] A method for improving a cell culturing rate at a time of producing cell aggregates which comprises using a copolymer containing a recurring unit derived from a monomer represented by the following formula (I): 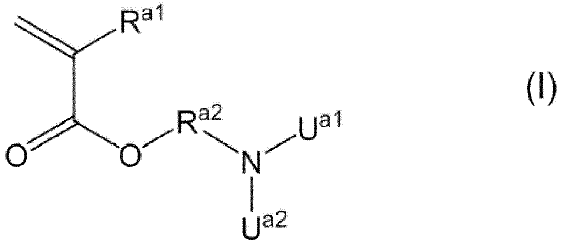 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
      and a recurring unit derived from a monomer represented by the following formula (II): 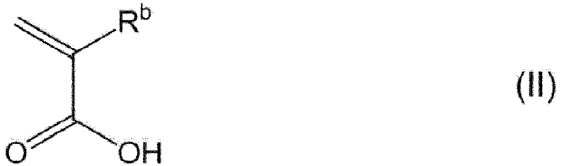 [wherein
   R^{b} represents a hydrogen atom or a linear or branched alkyl group having 1 to 5 carbon atoms]
   as a base coat agent for cell culture on a substrate having an ability to inhibit cell adhesion, which is carried out by lowering a molar ratio of the recurring unit derived from the formula (I) to the whole copolymer.
[10] A method for controlling a cell culturing rate at a time of producing cell aggregates which comprises using a copolymer containing a recurring unit derived from a monomer represented by a recurring unit derived from a monomer represented by the following formula (I): 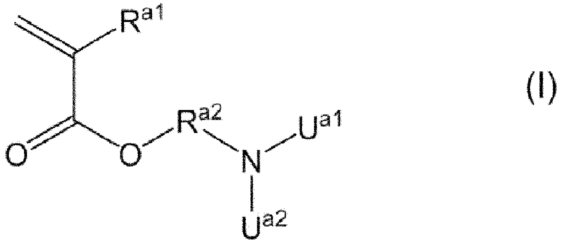 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
      and a recurring unit derived from a monomer represented by the following formula (II): 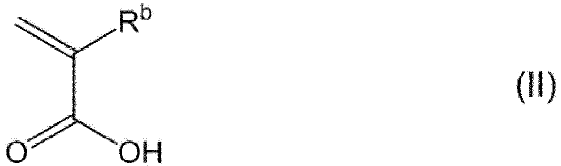 [wherein
   R^{b} represents a hydrogen atom or a linear or branched alkyl group having 1 to 5 carbon atoms]
   as a base coat agent for cell culture on a substrate having an ability to inhibit cell adhesion, which is carried out by changing a monomer ratio of the formula (I) and the formula (II) in the copolymer.
[11] A base coat agent for a cell culture container for promoting formation of cell aggregates which comprises a copolymer containing a recurring unit derived from a monomer represented by the following formula (I): 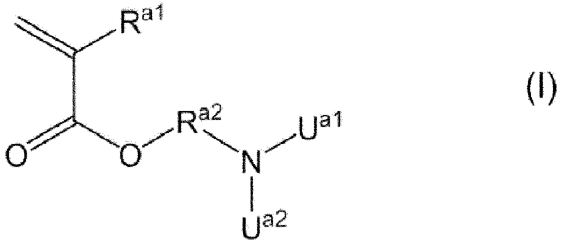 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
      and a recurring unit derived from a monomer represented by the following formula (II): 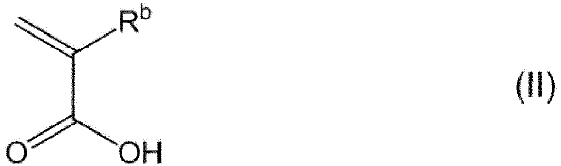 [wherein
   R^{b} represents a hydrogen atom or a linear or branched alkyl group having 1 to 5 carbon atoms].
[12] The base coat agent described in the above-mentioned [11], wherein a molar ratio of the recurring unit derived from the monomer represented by the above-mentioned formula (I) to a total of the recurring unit derived from the monomer represented by the above-mentioned formula (I) and the recurring unit derived from the monomer represented by the above-mentioned formula (II) is 99 mol% to 51 mol%.
[13] The base coat agent described in the above-mentioned [11] or [12], wherein the above-mentioned copolymer further contains a structural unit derived from a monomer represented by the following formula (III): 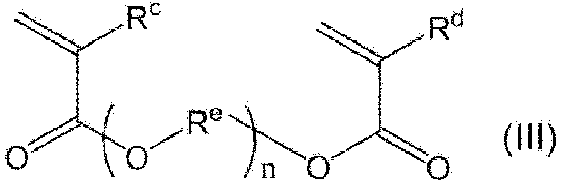 [wherein
   R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50].
[14] The base coat agent described in the any of above-mentioned [11] to [13], wherein promotion of formation of cell aggregates is that a completion time of forming cell aggregates after seeding cells is within 20 hours.

### EFFECTS OF THE INVENTION

As a result of intensive studies by the inventors, they have found that, by controlling the copolymer having a cationic functional group and an anionic functional group, specifically a molar ratio of the cationic functional group within a predetermined range (in particular, by reducing it in the predetermined range), when the copolymer is used as a base coat agent for the production of cell aggregates, the production rate of cell aggregates is markedly improved, and also, by changing the molar ratio of the cationic functional group and the anionic functional group adequately, the rate of producing cell aggregates can be controlled whereby they have completed the present invention.

According to the present invention, there are provided a substrate for producing cell aggregates capable of markedly shortening a producing time of cell aggregates as compared with the conventional method (for example, by a cell culture dish), specifically, a substrate for producing cell aggregates wherein a completion time for forming cell aggregates after seeding cells is within 20 hours and a method for producing the same, a method for producing cell aggregates, a method for improving a cell culturing rate at a time of producing cell aggregates, a method for controlling a cell culturing rate at a time of producing cell aggregates, and a base coat agent for a cell culture container for promoting formation of cell aggregates.

### BRIEF DESCRIPTION OF THE DRAWINGS

Fig. 1 is a microscopic photograph in which the states of cell adhesion and aggregation in Example 4 are observed.
Fig. 2 is a graph which compares a cell adhesion area and a cell culture time in Example 4.
Fig. 3 is a table which compares a cell adhesion area and a cell culture time in Example 4 (this is numerical data of Fig. 2).
Fig. 4 is a graph which compares a cell adhesion area and a DM molar ration based on 2-(dimethylamino)ethyl methacrylate (in the drawing, it is represented by DM) and methacrylic acid (in the drawing, it is represented by MA) in the copolymer in Example 4.

### EMBODIMENTS TO CARRY OUT THE INVENTION

### <Substrate for producing cell aggregates and method for producing the same>

The substrate for producing cell aggregates of the present application is a substrate for producing cell aggregates provided with a spot(s) comprising a copolymer containing a recurring unit derived from a monomer represented by the following formula (I): 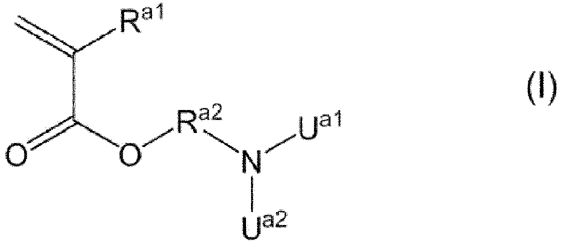 [wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms],
   and a recurring unit derived from a monomer represented by the following formula (II): 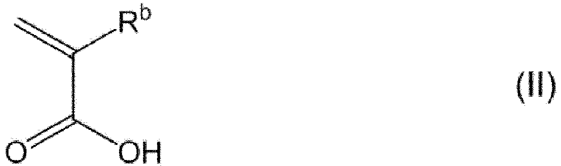 [wherein
R^{b} represents a hydrogen atom or a linear or branched alkyl group having 1 to 5 carbon atoms]
on a substrate having an ability to inhibit cell adhesion, wherein a completion time of forming cell aggregates after seeding cells is within 20 hours. Hereinafter, it is explained in order.

### (Copolymer)

The copolymer according to the present invention is a copolymer obtained by polymerizing
a cationic monomer represented by the following formula (I): 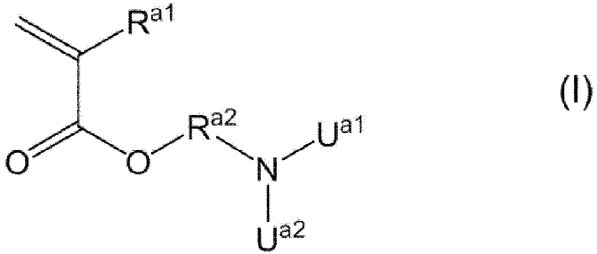 [wherein
U^{a1}, U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms]
   and an anionic monomer represented by the following formula (II): 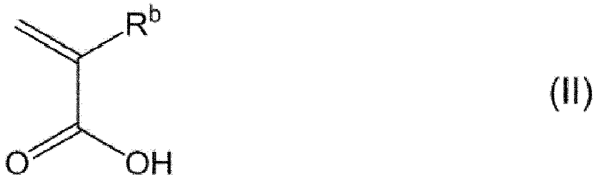 [wherein
R^{b} represents a hydrogen atom or a linear or branched alkyl group having 1 to 5 carbon atoms].

In the present specification, unless otherwise specifically defined, the "linear or branched alkyl group having 1 to 5 carbon atoms" may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group.

R^{a1} and R^{b} are preferably each independently selected from a hydrogen atom and a methyl group.

U^{a1} and U^{a2} are preferably each independently selected from a hydrogen atom, a methyl group, an ethyl group, an n-propyl group, an isopropyl group and an n-butyl group, preferably a methyl group or an ethyl group, and most preferably a methyl group.

In the present specification, unless otherwise specifically defined, as the "linear or branched alkylene group having 1 to 5 carbon atoms", there may be mentioned, for example, a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, etc. Among these, as R^{a2}, it is preferably selected from an ethylene group and a propylene group.

Accordingly, as the cationic monomer represented by the above-mentioned formula (I), there may be mentioned 2-N,N-dimethylaminoethyl methacrylate, N,N-dimethylaminomethyl methacrylate, etc., and preferably 2-N,N-dimethylaminoethyl methacrylate. As the anionic monomer represented by the above-mentioned formula (II), there may be mentioned acrylic acid, methacrylic acid, etc., and preferably methacrylic acid.

A molar ratio of the unit derived from the monomer represented by the formula (I)/the unit derived from the monomer represented by the formula (II) in the above-mentioned polymer is 99/1 to 51/49. It is preferably 98/2 to 60/40. It is preferably 95/5 to 70/30, and preferably 93/7 to 70/3.

If the molar ratio of the formula (II) is 50 or more, the anionic property of the copolymer becomes excessive and adhesive force of the cells is lowered.

### <Monomer having two or more carbon-carbon unsaturated bonds>

The above-mentioned copolymer may be a copolymer obtained by polymerizing the monomers represented by the formula (I)/the formula (II), and further a monomer having two or more carbon-carbon unsaturated bonds. The monomer having two or more carbon-carbon unsaturated bonds is specifically a monomer having two or more carbon-carbon double bonds, and there may be mentioned, for example, a polyfunctional acrylate compound, a polyfunctional acrylamide compound, a polyfunctional polyester or an isoprene compound, etc.

Preferred specific examples may be mentioned monomers represented by the following formulae (III) to (V).

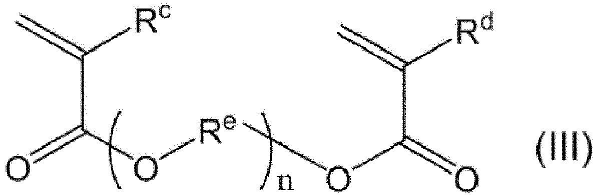

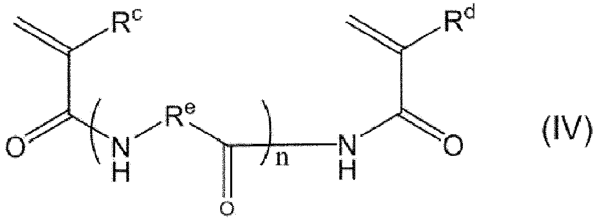

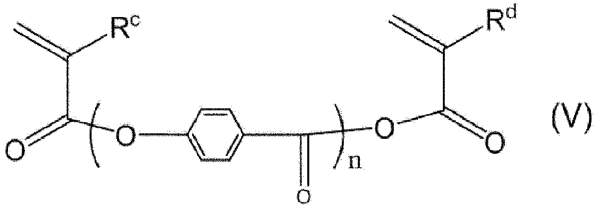

In the formulae, R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50. Among these, it is preferably a monomer represented by the formula (III).

A molar ratio of the monomer represented by the formulae (III) to (V) to the whole copolymer mentioned above is preferably 0 to 5.0%. It is further preferably 0 to 3.0%.

If the molar ratio of the formulae (III) to (V) is 5% or more, there is a fear of making production difficult due to gelation during production by becoming higher molecular weight due to excessive crosslinking.

R^{c} and R^{d} are each independently and preferably selected from a hydrogen atom and a methyl group.

R^{e} is preferably selected from a methylene group, an ethylene group and a propylene group, and most preferably an ethylene group.

n is a number of 1 to 50, n is preferably a number of 1 to 30, and n is preferably a number of 1 to 10.

The copolymer according to the present invention may be copolymerized with, as a further optional additional monomer component(s), an ethylenically unsaturated monomer, a polysaccharide(s) or a derivative thereof. Examples of the ethylenically unsaturated monomer may be mentioned one kind or two or more kinds of ethylenically unsaturated monomers selected from the group consisting of a (meth)acrylic acid and an ester thereof; vinyl acetate; vinylpyrrolidone; ethylene; vinyl alcohol; and a hydrophilic functional derivative thereof. Examples of the polysaccharide or a derivative thereof may be mentioned a cellulose-based polymer such as hydroxyalkyl cellulose (for example, hydroxyethyl cellulose or hydroxypropyl cellulose), etc., starch, dextran and curdlan.

The hydrophilic functional derivative refers to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or a divalent group of a compound having an amphoteric center of a quaternary ammonium type cation structure and an acidic anion structure and may be mentioned, for example, a phosphorylcholine group:

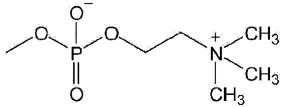

Examples of the ethylenically unsaturated monomer having such a structure may be mentioned 2-methacryloyloxyethyl phosphorylcholine (MPC), etc.

The amide structure means a group represented by the following formula: 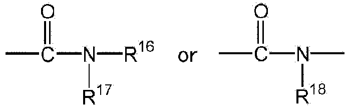 [here, R¹⁶, R¹⁷ and R¹⁸ are each independently a hydrogen atom or an organic group (for example, a linear or branched alkyl group, etc., which may be substituted, specifically, a methyl group, an isopropyl group, a hydroxymethyl group or a hydroxyethyl group, etc.)].

Examples of the ethylenically unsaturated monomer having such a structure may be mentioned (meth)acrylamide, N-isopropyl acrylamide, N-(hydroxymethyl) (meth)-acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is an alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group of the present invention, a quaternalized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 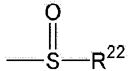 [here, R²² is an organic group (for example, an organic group having 1 to 10 carbon atoms, and preferably an alkyl group having 1 to 10 carbon atoms and having one or more hydroxyl groups, etc.)].

As a polymer having such a structure, there may be mentioned a copolymer disclosed in the publication of JP 2014-48278A, etc.

A difference between the value of mol% occupied by the monomer represented by the formula (II) based on the above-mentioned whole copolymer and the value of mol% occupied by the monomer represented by the formula (II) based on the total amount of the monomers charged during the preparation step is 0 to 10 mol%. The copolymer according to the present application has a small difference between the monomer charging ratio and the measured value of the produced copolymer by the production method mentioned later, and is 0 to 10 mol%. It is further preferably 0 to 8 mol%.

A number average molecular weight (Mn) of the above-mentioned copolymer is 20,000 to 1,000,000, and further preferably 50,000 to 800,000.

A ratio (Mw/Mn) of the weight average molecular weight (Mw) to the above-mentioned number average molecular weight (Mn) of the above-mentioned copolymer is 1.01 to 10.00, preferably 1.2 to 8.0, preferably 1.4 to 6.0, preferably 1.5 to 5.0, and particularly preferably 1.6 to 4.5.

The above-mentioned weight average molecular weight (Mw) and the number average molecular weight (Mn) can be obtained, for example, by Gel Filtration Chromatography described in Examples.

By utilizing the copolymer according to the present invention, it is possible to form cell aggregates by exfoliating after adhesion of the cells. Incidentally, the cell aggregates refer to a structural material formed as a result of aggregation of the cells, and a shape thereof is not limited to a spherical shape or a ring shape, etc. There is a merit in the point of adjusting a size of the cell aggregates according to the regulation of the adhesion area (cell aggregates having an optional size can be produced), etc., as compared with the cell aggregates produced on a conventional cell low-adhesion plate by non-adhesive culture.

### (Substrate)

The substrate for producing cell aggregates of the present invention can be produced by making the above-mentioned copolymer a base coat agent for a cell culture container by the method mentioned later, and applying it to the surface of the substrate and drying the same. Here, the "surface" refers to a surface in contact with the contents such as cells or cell culture medium.

A shape of the surface of the substrate may be a plane or may have unevenness, and a plane shape is preferable.

A material of the substrate may be mentioned, for example, glass, a metal, a metal containing compound or a semi-metal containing compound, activated charcoal or a resin. The metal may be mentioned a typical metal: an aluminum group element: Al, Ga, In; an iron group element: Fe, Co, Ni; a chromium group element: Cr, Mo, W, U; a manganese group element: Mn, Re; and a noble metal: Cu, Ag, Au, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned, for example, cellulose, cellulose triacetate (CTA), nitrocellulose (NC), cellulose to which dextran sulfate has been fixed, etc., and as the synthetic resin, there may be preferably used polyacrylonitrile (PAN), polyimide (PI), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), cycloolefin polymer (COP), polyvinyl chloride (PVC), polytetrafluoroethylene (PTFE), ultra-high molecular weight polyethylene (UHPE), polydimethylsiloxane (PDMS), acrylonitrile-butadienestyrene resin (ABS) or Teflon (Registered Trademark).

In the production of the substrate for producing cell aggregates of the present invention, it does not require a treatment at a high temperature at the time of applying the copolymer so as to exist at least a part of the surface of the substrate, so that a resin having low heat resistance, etc., can be also applicable.

A material of the substrate may be one kind or a combination of two or more kinds, and in the substrate for producing cell aggregates of the present application, for example, the substrate is preferably a substrate having flexibility so that it can be wound (roll system) like a belt conveyor in order to mass produce cell culture aggregates. As the material of the substrate used for the above-mentioned roll system, there may be mentioned a synthetic resin and a natural polymer.

Also, the substrate of the present application may be a substrate to be used for a so-called cell culture container. It may be mentioned petri dishes or dishes such as petri dishes generally used for cell culture, dishes for tissue culture, multi-dishes, flasks such as cell culture flasks, spinner flasks, bags such as plastic bags, Teflon (Registered Trademark) bags, culture bags, plates such as microplates, microwell plates, multiplates, multiwell plates, bottles such as chamber slides, tubes, trays, roller bottles, etc.

The copolymer of the present application can be produced by produced by a method known *per se* (for example, described in JP 2014-162865A and PCT/JP2019/032785).

### (Base agent)

By mixing a water-containing solution with the above-mentioned copolymer by a method known *per se,* a base coat agent for cell culture container of the present invention can be produced. The base coat agent of the present invention is useful for promoting formation of cell aggregates. Promoting formation of cell aggregates means a time of completion of formation of cell aggregates after seeding cells is within 20 hours as will be mentioned later.

The water-containing solution may be mentioned water, a salt-containing aqueous solution such as a physiological saline or a phosphate buffer solution, etc., or a mixed solvent in which water or the salt-containing aqueous solution and an alcohol are combined. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, and these may be used alone or may be used a mixed solvent in combination thereof.

Further, to the base coat agent may be added, in addition to the above-mentioned copolymer and the solvent, other substance(s) within the range which do not impair the properties of the resulting base film, if necessary. As the other substance(s), there may be mentioned pH adjusting agents, crosslinking agents, preservatives, surfactants, primers that enhance adhesiveness to the container or the substrate, fungicides and saccharides, etc.

### (Spot)

In a spot comprising the above-mentioned copolymer, preferably a plurality of spots (coating film) provided by the substrate for producing cell aggregates of the present invention, a ratio of the total area of the spots to the surface area of the substrate is not particularly limited, and for example, it is 30% or more, and a diameter of each spot is, for example, 50 to 5,000 µm, and a distance between the spots is, for example, 30 to 1,000 µm. A shape of the spot is not particularly limited. For example, it is a substantially circular or a rectangular shape, and it is preferably a substantially circular spot.

A ratio of the total area of the spots to the surface area of the substrate, and a diameter of each spot and a distance between the spots can be optionally selected from a predetermined range according to the kinds of cells to be used and the substrate, a desired size of the cell aggregates, etc., the ratio of the total area of the spots to the surface area of the substrate is preferably 30% or more, 40% or more and 50% or more, and preferably 99% or less, the diameter of each spot is 50 to 5,000 µm, and preferably 300 to 3,000 µm, and the distance between the spots is 30 to 1,000 µm, and preferably 100 to 500 µm.

In the present invention, a plurality of spheroids having uniform size can be formed with one substrate (container) at once by arranging independent micro-sized regions (spots) to which cells can adhere with such a high density, preferably regularly, on a substrate having an ability to inhibit cell adhesion.

The spot comprising the above-mentioned copolymer can be formed by applying the copolymer, preferably the above-mentioned base coat agent for a cell culture container. As the system of applying the base coat agent, there may be employed, for example, an inkjet method, a screen printing method, a slit coating method, a roll-to-roll method, etc., and preferably carried out by a printing technique of the inkjet method or the screen printing method.

As another applying method, for example, there may be employed a method in which a substrate in which the non-formed portion of the spot is protected is immersed in the above-mentioned base coat agent as the case may be, a base coat agent is added to the substrate (container) in which the non-formed portion of the spot is protected as the case may be and allowed to stand for a predetermined time, etc., and in the case of a substrate, or a cell culture container as an embodiment, it is carried out by the method in which the base coat agent is added to a container the non-formed portion of the spot is protected as the case may be and allowed to stand for a predetermined time. Addition can be carried out by adding, for example, 0.5 to 1-fold amount of the base coat agent based on the whole volume of the container using a syringe, etc. Allowing to stand can be practiced by optionally selecting a time and temperature depending on the material of the container or the substrate, the kind of the base coat agent for cell culture, and is practiced, for example, 1 minute to 24 hours, preferably 5 minutes to 3 hours at 10 to 80°C. According to the procedure, the substrate for producing cell aggregates can be produced.

Also, the spots on the surface of the substrate obtained by such a method can be used as a substrate for producing cell aggregates without subjecting to the drying step as such, or after washing using water or a medium of the sample applied to cell culture (for example, water, buffer solution, medium, etc.).

That is, after forming the spots on the surface of the above-mentioned substrate, within 48 hours, preferably within 24 hours, further preferably within 12 hours, further preferably within 6 hours, further preferably within 3 hours, further preferably within 1 hour, without subjecting to a drying step as such, or after washing using water or a medium of a sample applied to cell culture (for example, water, buffer solution, medium, etc., particularly preferably medium (for example, DMEM medium (Dulbecco's modified eagle medium)), it can be used as a substrate for producing cell aggregates.

The substrate for producing cell aggregates may be subjected to a drying step. The drying step is carried out under atmosphere or under vacuum, preferably at a temperature in the range of -200°C to 200°C. According to the drying step, by removing the solvent in the above-mentioned base coat agent, it completely adheres to the substrate.

The spots can be also formed, for example, by drying at room temperature (10°C to 35°C, preferably 20°C to 30°C, for example, 25°C), but in order to form the spots more quickly, for example, it may be dried at 40°C to 50°C. If the drying temperature is lower than -200°C, a refrigerant which is not common must be used so that it is not versatile, and it takes a long time to dry due to solvent sublimation so that efficiency is bad. If the drying temperature exceeds 200°C, thermal decomposition of the copolymer occurs. A more preferable drying temperature is 10°C to 180°C, and a more preferable drying temperature is 20°C to 150°C.

The substrate for producing cell aggregates of the present application is produced through the above simple and easy steps.

Also, in order to eliminate impurities, unreacted monomers, etc., remained in the spots (coating film), a step of washing with at least one kind of a solvent selected from water and an aqueous solution containing an electrolyte(s) may be carried out. Washing is desirably running water washing or ultrasonic washing, etc. The above-mentioned water and an aqueous solution containing an electrolyte(s) may be a material heated in the range of, for example, 40°C to 95°C. The aqueous solution containing an electrolyte(s) is/are preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and alcohol, etc.

Regarding the film thickness of the spot (coating film) of the present application, the maximum film thickness and the minimum film thickness are in the range of 1 to 1,000 nm, and preferably in the range of 5 to 500 nm.

The substrate for producing cell aggregates of the present application is produced by a substrate having an ability to inhibit adhesion of cells. The substrate may be subjected to a treatment of inhibiting adhesion of cells before formation of the above-mentioned spots (coating film). The substrate having an ability to inhibit adhesion of cells can be produced, for example, through a step of applying a known composition for forming a coating film having an ability of inhibiting adhesion of cells. As the composition for forming a coating film having an ability of inhibiting adhesion of cells, it is preferable to contain steps of applying a composition for forming a coating film which comprises a copolymer (P) having a recurring unit containing the group represented by the following formula (a) and a recurring unit containing the group represented by the following formula (b): 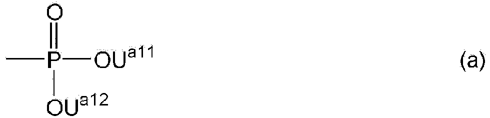 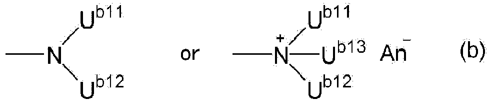 [wherein
U^{a11}, U^{a12}, U^{b11}, U^{b12} and U^{b13} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion]
and a solvent on the surface of a container or a substrate and drying. The above-mentioned coating film may be comprised at least a part of the surface of the substrate, and it is preferable that it is applied over the entire surface for producing cell aggregates (that is, the surface where the spots of the present application are present), or over the entire surface of the substrate.

The linear or branched alkyl group having 1 to 5 carbon atoms is the same as mentioned above.

As the above-mentioned composition for forming a coating film, for example, a composition for forming a coating film described in WO2014/196650 can be used.

The applying method of the above-mentioned composition for forming a coating film is not particularly limited, and a usual applying method such as spin coating, dip coating, solvent casting method, etc., is used.

The drying step of the above-mentioned coating film is carried out under atmosphere or under vacuum at a temperature in the range of -200°C to 180°C. According to the drying step, the solvent in the above-mentioned composition for forming a coating film is removed, and the formula (a) and the formula (b) in the above-mentioned copolymer form an ionic bond and are completely adhered to the substrate.

The above-mentioned coating film can be also formed, for example, by drying at room temperature (10°C to 35°C, for example, 25°C), but in order to form the coating film more quickly, it may be dried at, for example, 40°C to 50°C. In addition, a drying step at an extremely low temperature to a low temperature (around -200°C to -30°C) by a freeze-drying method may be used. Freeze-drying is called to as vacuum freeze-drying, which is a method of usually cooling what is desired to be dried with a refrigerant and removing the solvent by sublimation in a vacuum state. A general refrigerant used in freeze-drying may be mentioned a mixed medium of dry ice and methanol (-78°C), liquid nitrogen (-196°C), etc.

If the drying temperature is -200°C or lower, a refrigerant which is not common must be used so that it is not versatile, and it takes a long time to dry due to solvent sublimation so that efficiency is bad. If the drying temperature is 200°C or higher, the ionic bonding reaction on the surface of the coating film proceeds excessively and the surface loses its hydrophilicity whereby the ability to inhibit adhesion of biological substances is not exhibited. A more preferable drying temperature is 10°C to 180°C, and a more preferable drying temperature is 25°C to 150°C.

After drying, it is desirable to carry out washing such as running water washing or ultrasonic washing, etc., with one or more solvents selected from water and an aqueous solution containing an electrolyte(s) in order to eliminate impurities, unreacted monomers, etc., remained on the coating film, and further to adjust the ion balance of the copolymer in the film. The above-mentioned water and an aqueous solution containing an electrolyte(s) may be a material heated in the range of, for example, 40°C to 95°C. The aqueous solution containing an electrolyte(s) is preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and alcohol, etc. Even when a biological substance is attached to the formed coating film, it can be easily removed by washing with water, etc., thereafter, and the surface of the substrate on which the above-mentioned coating film is formed has an ability to inhibit adhesion of the biological substance.

A film thickness of the above-mentioned coating film is preferably 5 to 1,000 nm, and further preferably 5 to 500 nm.

Having an ability to inhibit cell adhesion means that, for example, a relative absorbance (WST O.D. 450 nm) (%) ((absorbance of Example (WST O.D.450 nm))/(absorbance of Comparative Example (WST O.D. 450 nm))) when compared with no coating film or without cell low-adhesion treatment by the fluorescence microscope carried out by the method described in, for example, Example of WO2016/093293 is 50% or less, preferably 30% or less, and further preferably 20% or less.

Also, as the substrate having an ability to inhibit adhesion of cells, a commercially available cell culture dish subjected to cell low-adhesion treatment, a cell culture container having an ability to inhibit adhesion of cells, etc., may be used. For example, a cell culture container described in JP 2008-61609A can be used, but the invention is not limited to this.

The coating film having an ability to inhibit adhesion of cells according to the present invention, those in which an ethylenically unsaturated monomer, or a polysaccharide or a derivative thereof is copolymerized may be used. Examples of the ethylenically unsaturated monomer may be mentioned one kind or two or more kinds of ethylenically unsaturated monomer(s) selected from the group consisting of (meth)acrylic acid and an ester thereof; vinyl acetate; vinylpyrrolidone; ethylene; vinyl alcohol; and a hydrophilic functional derivative thereof. Examples of the polysaccharide or a derivative thereof may be mentioned cellulose-based polymers such as hydroxyalkyl cellulose (for example, hydroxyethyl cellulose or hydroxypropyl cellulose), etc., starch, dextran and curdlan.

The hydrophilic functional derivative refers to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or a divalent group of a compound having an amphoteric center of a quaternary ammonium type cation structure and an acidic anion structure and may be mentioned, for example, a phosphorylcholine group:

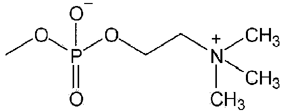

Examples of the ethylenically unsaturated monomer having such a structure may be mentioned 2-methacryloyloxyethyl phosphorylcholine (MPC), etc.

The amide structure means a group represented by the following formula: 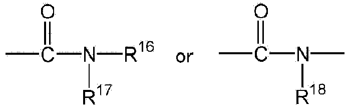 [here, R¹⁶, R¹⁷ and R¹⁸ are each independently a hydrogen atom or an organic group (for example, a methyl group, a hydroxymethyl group or a hydroxyethyl group, etc.)]. Examples of the ethylenically unsaturated monomer having such a structure may be mentioned (meth)acrylamide, N-(hydroxymethyl) (meth)acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is an alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group of the present invention, a quaternalized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 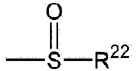 [here, R²² is an organic group (for example, an organic group having 1 to 10 carbon atoms, and preferably an alkyl group having 1 to 10 carbon atoms and having one or more hydroxyl groups, etc.)].

As the polymer having such a structure, there may be mentioned a copolymer disclosed in the publication of JP 2014-48278A, etc.

### (Cell)

A cell in the present invention is the most basic unit constituting an animal or a plant, and has a cytoplasm and various kinds of organelles inside the cell membrane as its elements. At this time, the nucleus containing DNA may or may not be contained inside the cell. For example, the animal-derived cells in the present invention include germ cells such as sperm and egg cells, etc., somatic cells constituting the living body, stem cells (pluripotent stem cells, etc.), precursor cells, cancer cells separated from the living body, cells separated from the living body and have acquired immortalization ability and are stably maintained in vitro (cell lines), cells separated from the living body and genetic modification has artificially been done, and cells separated from the living body and nuclei of which have artificially been exchanged, etc. Examples of the somatic cells constituting the living body are not limited to the following, and include fibroblast, bone marrow cells, B lymphocytes, T lymphocytes, neutrophils, red blood cells, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells (for example, CD34-positive cells derived from umbilical cord blood), and mononuclear cells, etc. The somatic cells include, for example, cells collected from an optional tissue such as skin, kidney, spleen, adrenal, liver, lung, ovary, pancreas, uterus, stomach, colon, small intestine, large intestine, bladder, prostate, testis, thoracic gland, muscle, connective tissue, bone, cartilage, blood vessel tissue, blood (including umbilical cord blood), bone marrow, heart, eye, brain or nerve tissue, etc. Further, the somatic cells include cells differentiated from or derived from the stem cells or precursor cells.

The stem cells are cells that have the ability to replicate themselves and differentiate into other multi-lineage cells, and examples thereof include, but not limited to the following, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells, etc. As the pluripotent stem cells, among the above-mentioned stem cells, there may be mentioned ES cells, embryonic germline stem cells and iPS cells. The precursor cells are cells in the process of differentiating from the above-mentioned stem cells into specific somatic cells or germ cells. The cancer cells are cells that are derived from somatic cells and have acquired infinite proliferative capacity. The cell line is a cell that has acquired infinite proliferative capacity by artificial manipulation *in vitro.* Among these, adherent cells that survive and proliferate by adhering to the scaffold are preferable, and fibroblast or stem cells are more preferable.

### (Formation time of cell aggregates after seeding cells)

The substrate for producing cell aggregates of the present application is characterized in that the cell agglutination completion time after cell seeding is within 20 hours. In usual procedure, the production of cell aggregates takes several days, but it can be carried out in a short time. The above-mentioned completion time is within 20 hours, and may be within 15 hours, within 10 hours, within 9 hours, within 8 hours, within 7 hours, within 6 hours, or within 5 hours.

### <Method for producing cell aggregates>

The method for producing cell aggregates of the present application is a method for producing cell aggregates which comprises using a copolymer containing a recurring unit derived from a monomer represented by the following formula (I): 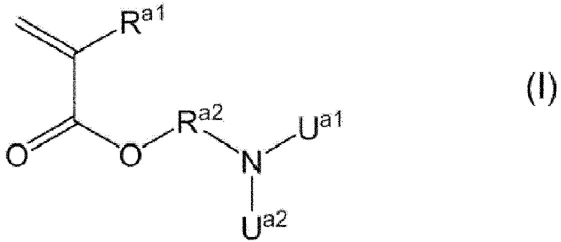 [wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms], and a recurring unit derived from a monomer represented by the following formula (II): 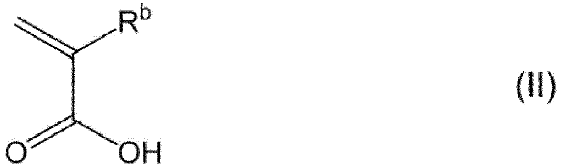 [wherein
R^{b} represents a hydrogen atom or a linear or branched alkyl group having 1 to 5 carbon atoms] as a base coat agent for cell culture on a substrate having an ability to inhibit cell adhesion, wherein a completion time of forming cell aggregates after seeding cells is within 20 hours. The meanings and preferred embodiments of the symbols described in the formula (I) and the formula (II) are as mentioned above.

The above-mentioned base coat agent for cell culture is shaped into the spot state on the substrate for producing cell aggregates of the present application as mentioned above, and culturing the above-mentioned cells by a known method *per se* to produce cell aggregates.

Specifically, The method for producing cell aggregates of the present application comprises a step of seeding cells on a substrate for producing cell aggregates of the present invention, which is provided with spots comprising a copolymer containing the recurring unit derived from the monomer represented by the above-mentioned formula (I) and the recurring unit derived from the monomer represented by the above-mentioned formula (II) on a substrate having an ability to inhibit cell adhesion, and a step of culturing the cells after seeding thereof to form cell aggregates within 20 hours.

### <Method for improving production rate of cell aggregates, and method for controlling production rate of cell aggregates>

A method for improving a production rate of cell aggregates of the present application is a method for improving a cell culturing rate at the time of producing cell aggregates, which comprises using a copolymer containing a recurring unit derived from a monomer represented by the following formula (I): 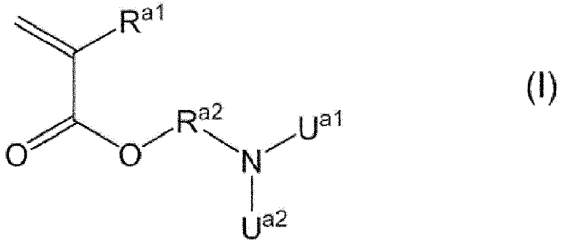 [wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms], and a recurring unit derived from a monomer represented by the following formula (II): 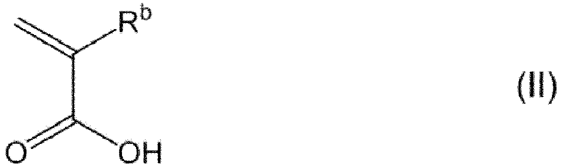 [wherein
R^{b} represents a hydrogen atom or a linear or branched alkyl group having 1 to 5 carbon atoms] as a base coat agent for cell culture, that is a method for improving a cell culturing rate at the time of producing cell aggregates, or a method for controlling a cell culturing rate at the time of producing cell aggregates which is to carry out by lowering a molar ratio of the formula (I) to the whole copolymer. The meanings and preferred embodiments of the symbols described in the formula (I) and the formula (II) are as mentioned above.

Specifically, by making a molar ratio of the recurring unit derived from the monomer represented by the above-mentioned formula (I) less in the range of 99 mol% to 51 mol% to the total of the recurring unit derived from the monomer represented by the above-mentioned formula (I) and the recurring unit derived from the monomer represented by the above-mentioned formula (II), a cell culturing rate can be improved. The reason for this can be considered that is an amount of the cation portion derived from the monomer represented by the above-mentioned formula (I) of the copolymer of the present application increases, cell adhesion ability increases, but conversely, if the adhesion force is too strong, the cells do not peel off while adhering to the copolymer of the present application and cell aggregates cannot be formed, and to the contrary, if an amount of the anion component derived from the above-mentioned formula (II) is too much, the cells do not adhere and adhesion culture for the production of cell aggregates of the present application cannot be achieved. A ratio of cation/anion for controlling a suitable time for the production of cell aggregates is considered to have a suitable range depending on the kind of the cells used for the production of cell aggregates. According to the method of the present application, it is possible to improve a production rate of cell aggregates, that is, to shorten the production time of cell aggregates, or depending on the kind of the cells, it is possible to set an optimum production time of cell aggregates (that is, to control the production time of cell aggregates).

### EXAMPLES

Hereinafter, the present invention will be explained in more detail by referring to Examples, but the present invention is not limited to these.

### <Measurement method of molecular weight>

The weight average molecular weight shown in the following Synthetic Examples is the result by Gel Filtration Chromatography (hereinafter abbreviated to as GFC).

### (Measurement conditions)

- Device: HLC-8320GPC (manufactured by Tosoh Corporation)
- GFC column: TSKgel G 6000 + 3000 PWXL-CP
- Flow rate: 1.0 mL/min
- Eluent: Water/organic mixed solvent containing a salt
- Column temperature: 40°C
- Detector: RI
- Injection concentration: Polymer solid content of 0.05% by mass
- Injection amount: 100 µL
- Calibration curve: Cubic approximation curve
- Standard sample: Polyethylene oxide (available from Agilent Technologies) × 10 kinds

### <Synthetic Example 1> Production (1) of copolymer used as base coat agent for cell culture by thermal polymerization

To 10.00 g of 2-(dimethylamino)ethyl methacrylate (hereinafter sometimes abbreviated to as "DM") (available from Tokyo Chemical Industry Co., Ltd.) was added 31.46 g of ethanol, and the mixture was sufficiently dissolved. Then, 0.01 g of dimethyl 1,1'-azobis(1-cyclohexanecarboxylate) (product name; VE-073, available from FUJIFILM Wako Pure Chemical Corporation), 0.48 g of methacrylic acid (hereinafter sometimes abbreviated to as "MA") (available from Tokyo Chemical Industry Co., Ltd.) and 0.21 g of polyethylene glycol dimethacrylate (hereinafter sometimes abbreviated to as "PEGDMA") (n=about 4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a copolymer. The weight average molecular weight of this copolymer according to GFC was 770,000, and the polydispersity was 4.1 (Synthetic Example polymer 1).

### <Synthetic Example 2> Production (2) of copolymer used as base coat agent for cell culture by thermal polymerization

To 10.00 g of 2-(dimethylamino)ethyl methacrylate (available from Tokyo Chemical Industry Co., Ltd.) was added 43.90 g of ethanol, and the mixture was sufficiently dissolved. Then, 0.01 g of dimethyl 1,1'-azobis(1-cyclohexanecarboxylate) (product name; VE-073, available from FUJIFILM Wako Pure Chemical Corporation), 0.97 g of methacrylic acid (available from Tokyo Chemical Industry Co., Ltd.) and 0.22 g of polyethylene glycol dimethacrylate (n=about 4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a copolymer. The weight average molecular weight of this copolymer according to GFC was 663,000, and the polydispersity was 3.6 (Synthetic Example polymer 2).

### <Synthetic Example 3> Production (3) of copolymer used as base coat agent for cell culture by thermal polymerization

To 10.00 g of 2-(dimethylamino)ethyl methacrylate (available from Tokyo Chemical Industry Co., Ltd.) was added 45.51 g of ethanol, and the mixture was sufficiently dissolved. Then, 0.01 g of dimethyl 1,1'-azobis(1-cyclohexanecarboxylate) (product name; VE-073, available from FUJIFILM Wako Pure Chemical Corporation), 1.37 g of methacrylic acid (available from Tokyo Chemical Industry Co., Ltd.) and 0.23 g of polyethylene glycol dimethacrylate (n=about 4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a copolymer. The weight average molecular weight of this copolymer according to GFC was 856,000, and the polydispersity was 4.4 (Synthetic Example polymer 3).

### <Synthetic Example 4> Production (4) of copolymer used as base coat agent for cell culture by thermal polymerization

To 10.00 g of 2-(dimethylamino)ethyl methacrylate (available from Tokyo Chemical Industry Co., Ltd.) was added 67.07 g of ethanol, and the mixture was sufficiently dissolved. Then, 0.01 g of dimethyl 1,1'-azobis(1-cyclohexanecarboxylate) (product name; VE-073, available from FUJIFILM Wako Pure Chemical Corporation), 1.83 g of methacrylic acid (available from Tokyo Chemical Industry Co., Ltd.) and 0.24 g of polyethylene glycol dimethacrylate (n=about 4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a copolymer. The weight average molecular weight of this copolymer according to GFC was 508,000, and the polydispersity was 2.9 (Synthetic Example polymer 4).

### <Synthetic Example 5> Production (5) of copolymer used as base coat agent for cell culture by thermal polymerization

To 10.00 g of 2-(dimethylamino)ethyl methacrylate (available from Tokyo Chemical Industry Co., Ltd.) was added 70.00 g of ethanol, and the mixture was sufficiently dissolved. Then, 0.01 g of dimethyl 1,1'-azobis(1-cyclohexanecarboxylate) (product name; VE-073, available from FUJIFILM Wako Pure Chemical Corporation), 2.35 g of methacrylic acid (available from Tokyo Chemical Industry Co., Ltd.) and 0.25 g of polyethylene glycol dimethacrylate (n=about 4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a copolymer. The weight average molecular weight of this polymer according to GFC was 578,000, and the polydispersity was 3.6 (Synthetic Example polymer 5).

### <Example 1> (Composition analysis by 1H-NMR measurement of polymer)

The nuclear magnetic resonance spectra (NMR) of Synthetic Example polymers 1 to 5 were measured using a nuclear magnetic resonance apparatus (manufactured by BRUKER Corporation, model number: ASCEnd500), and heavy water (D₂O) as a standard substance. In the following, representative peaks common to Synthetic Example polymer 1 to Synthetic Example polymer 5 are shown.

¹H-NMR (in D₂O) δ 0.8-1.2 (br, -CH2-C(CH3)-), 1.6-2.0 (br, -CH2-C(CH3)-), 2.2-2.4 (br, -N(CH3)2), 2.5-2.7 (br, -CH2-N(CH3)2), 4.0-4.2 (br, -O-CH2-).

Here, from the number of protons (in the case of the homopolymer of DM, 3 per one molecule of the monomer) A of the methyl group -CH2-C(CH3)- (δ 0.8-1.2) in the main chain, and the number of methyl protons (in the case of the homopolymer of DM, 2 per one molecule of the monomer) B of the -O-CH2- group (δ 4.0-4.2) in the side chain, the ratio of the number of the functional groups of the amino groups possessed by the side chain to the number of the functional groups of the carboxyl groups of the side chain was calculated.

As a result, with regard to Synthetic Examples 1 to 6 synthesized by thermal polymerization, it became 92/8 in the case of Synthetic Example 1, 81/19 in the case of Synthetic Example 2, 76/24 in the case of Synthetic Example 3, 72/28 in the case of Synthetic Example 4, and 70/30 in the case of Synthetic Example 5. Detailed results are shown in Table 1.

With regard to the polymers synthesized in Synthetic Examples 1 to 5, each became a high molecular weight product having 500,000 or more of the weight average molecular weight Mw. In addition, the range of the molecular weight distribution (PDI) was a relatively wide distribution of 2.9 or more. This is because a polyfunctional crosslinking agent is contained in the copolymer, whereby a polymer having reduced dissolution and excellent in coatability can be produced.

**[Table 1]**

| | Charged ratio | Compositional ratio (NMR in D2O) | Mw ( x 10^4) | Mw/Mn |
|---|---|---|---|---|
| | DM/MA/PEGDMA | DM/MA | | |
| Synthetic Example 1 | 92/8/2 | 92/8 | 77 | 4.1 |
| Synthetic Example 2 | 85/15/2 | 81/19 | 66 | 3.6 |
| Synthetic Example 3 | 80/20/2 | 76/24 | 86 | 4.4 |
| Synthetic Example 4 | 75/25/2 | 72/28 | 51 | 2.9 |
| Synthetic Example 5 | 70/30/2 | 70/30 | 58 | 3.6 |

### <Example 2> Production of polymer aqueous solution for producing base coat agent for cell culture

Each 0.01 g of Synthetic Example polymers 1 to 5 was dissolved by adding 100 g of sterilized water to produce base coat agents 1 to 5 of cell culture.

### <Example 3: Preparation of plate for forming cell aggregates>

### (3-1. Preparation of cell low-adhesion plate)

According to the production method described in Example 30 of WO2014/196650, a coating solution was prepared from a copolymer-containing varnish. The prepared coating solution was added to a well of a 24-well cell culture plate (manufactured by BD Biosciences, #351147) so as to be 2 mL (solid content of 0.5% by mass)/well, and after allowing to stand at room temperature for 1 hour, excess coating solution was removed. Thereafter, 3 mL of sterilized water was added per each well, and removed to carry out washing. Similarly, washing was further carried out twice, and it was dried at room temperature for 30 minutes to obtain a cell low-adhesion plate.

### (Production of base coat agent for cell culture, and preparation of polymer coating plate used as base coat film for cell culture)

### (3-2) Preparation of petri dish for forming cell aggregates by ink jet

Using an ink jet device (manufactured by MICROJET Co., Ltd., model number: LaboJet-600) and ink jet head (model number: 500-S-C), to cell low-adhesion petri dish prepared as mentioned above was applied each 50 nL of the base coat agents 1 to 5 for cell culture in a spot state. It was dried at room temperature for 30 minutes to form a film to obtain a petri dish for forming cell aggregates.

### <Example 4: Confirmation test of forming rate of cell aggregates>

### (4-1. Preparation of cells)

Mouse fetal fibroblasts (C3H10T1/T2 cells: available from DS Pharma Biomedical Co., Ltd.) were used as cells. For culturing the cells, a medium in which, to a BME medium (available from Gibco) which became a basal medium, were added so as to be 10% of FBS (available from Sigma-Aldrich) and 1% of Glutamine/Penicillin/Streptomycin (available from Gibco) was used. The cells were statically cultured for 2 days or more in a petri dish (medium 10 mL) having a diameter of 10 cm while maintaining a 5% carbon dioxide concentration in a 37°C/CO₂ incubator. Subsequently, the present cells were washed with 3 mL of a PBS solution (available from FUJTFILM Wako Pure Chemical Corporation), then, 3 mL of a trypsin-EDTA solution (available from PromoCell GmbH) was added thereto, and the cells were allowed to stand at room temperature for 3 minutes to exfoliate the cells. The cells were recovered by adding 7 mL of the above-mentioned medium. This suspension was centrifuged (manufactured by TOMY SEIKO Co., Ltd., model number LC-230, 200 × g/3 min, room temperature), and then, the supernatant was removed, and the above-mentioned medium was added to prepare a cell suspension.

### (4-2. Test for forming cell aggregates)

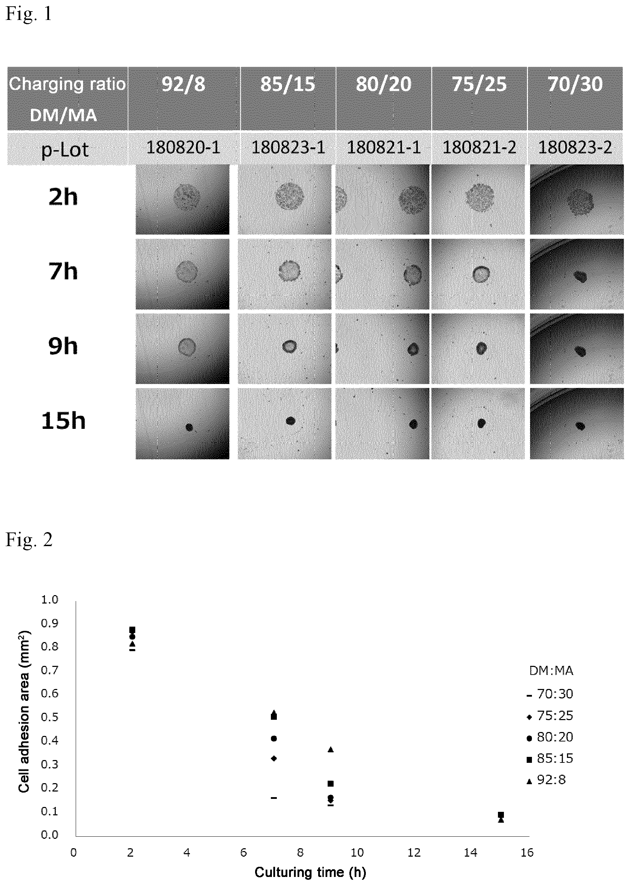

To the plate prepared in the above-mentioned Example 3 was added each 1 mL of each cell suspension so as to be 5.0 × 10⁵ cells/cm² to the plate. Thereafter, it was allowed to stand in a 37°C/CO₂ incubator for 2 hours while maintaining a 5% carbon dioxide concentration. After allowing to stand, non-adherent cells and the medium were removed, and it was washed with PBS to leave adherent cells alone on the wells. After washing, 1 mL/well of a new medium was added thereto, and the states of cell adhesion and aggregation were observed using an inverted research microscope IX83 (manufactured by Olympus Corporation) while culturing with lapse of time. As shown in Fig. 1, at the time after 2 hours, the cells were uniformly adhered only to the portion where the base coat agent was spotted regardless of the ratio of DM/MA. In addition, the rate of cell aggregation differed with a lapse of time depending on the ratio of DM/MA.

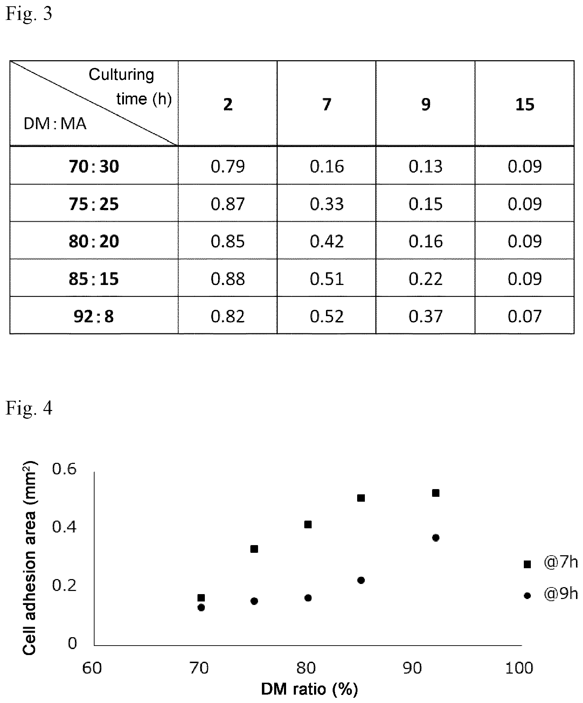

The cell adhesion area at each time was quantitated using the image analysis software ImageJ. As shown in Fig. 2 and Fig. 3, at the time after 2 hours, it was substantially constant regardless of the ratio of DM/MA, but at the time after 7 hours and after 9 hours, the cell adhesion area became large as the ratio of DM increased. In addition, at the time after 15 hours, aggregation was completely completed in all compositions, and spheroid diameters became substantially constant size regardless of the composition. From the above, according to the present invention, by changing the ratio of DM/MA under the conditions of constant concentration of the base coat agent and constant applying amount, it is possible to control the aggregation rate while maintaining the initial adhesion area of cells and the size of the cell aggregates finally formed constant.

### UTILIZABILITY IN INDUSTRY

As mentioned above, by controlling the molar ratio of the cationic functional group of the copolymer within a predetermined range, when the copolymer is used as a base coat agent for the production of cell aggregates, it is clarified that the rate of producing cell aggregates is markedly improved, and also, by changing the molar ratio of the cationic functional group to the anionic functional group appropriately, the rate of producing cell aggregates can be controlled. Therefore, according to the present invention, it can be provided a substrate for producing cell aggregates, which can significantly shorten the production time of cell aggregates, specifically, a substrate for producing cell aggregates (for example, a petri dish, a dish or a plate for cell culture), etc., in which the completion time of forming cell aggregates after seeding cells is within 20 hours.

## Claims

1. A substrate for producing cell aggregates provided with a spot(s) comprising a copolymer containing a recurring unit derived from a monomer represented by the following formula (I): 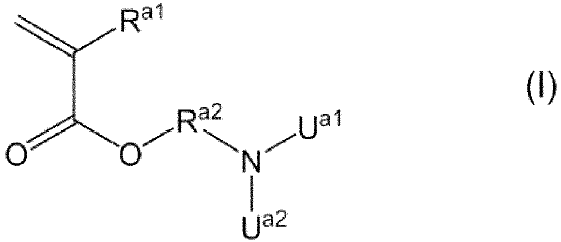 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 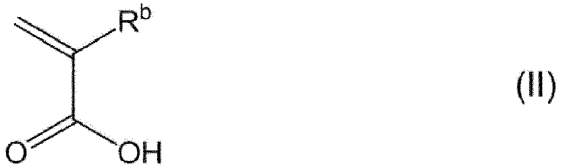 wherein
R^{b} represents a hydrogen atom or a linear or branched alkyl group having 1 to 5 carbon atoms,
on a substrate having an ability to inhibit cell adhesion, wherein a completion time of forming cell aggregates after seeding cells is within 20 hours.

2. The substrate for producing cell aggregates according to claim 1, wherein a molar ratio of the recurring unit derived from the monomer represented by the formula (I) to a total of the recurring unit derived from the monomer represented by the formula (I) and the recurring unit derived from the monomer represented by the formula (II) is 99 mol% to 51 mol%.

3. The substrate for producing cell aggregates according to claim 1 or 2, wherein the copolymer further contains a structural unit derived from a monomer represented by the following formula (III): 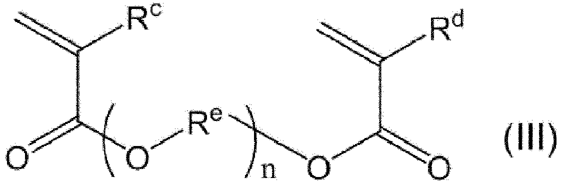 wherein
R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50.

4. The substrate for producing cell aggregates according to any one of claims 1 to 3, wherein the cells are stem cells or fibroblasts.

5. The substrate for producing cell aggregates according to any one of claims 1 to 4, wherein the substrate having an ability to inhibit cell adhesion comprises a coating film which comprises a copolymer (P) having a recurring unit containing a group represented by the following formula (a) and a recurring unit containing a group represented by the following formula (b): 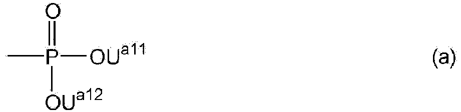 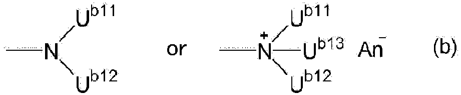 wherein
U^{a11}, U^{a12}, U^{b11}, U^{b12} and U^{b13} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion,
on at least a part of a surface thereof.

6. A method for producing a substrate for producing cell aggregates, comprising steps of applying a copolymer containing a recurring unit derived from a monomer represented by the following formula (I): 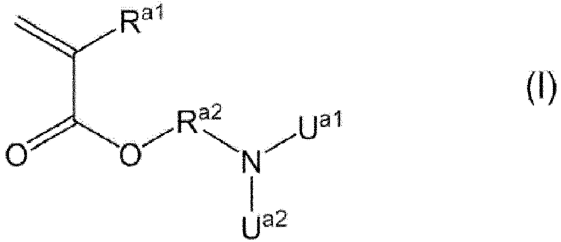 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 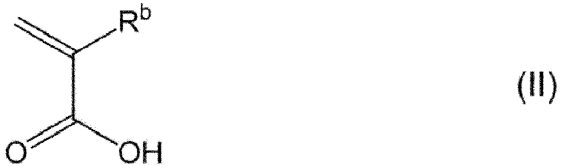 wherein
R^{b} represents a hydrogen atom or a linear or branched alkyl group having 1 to 5 carbon atoms,
in a plurality of spot states to a substrate having an ability to inhibit cell adhesion, and then, drying the same, wherein a completion time of forming cell aggregates after seeding cells is within 20 hours.

7. The method for producing a substrate for producing cell aggregates according to claim 6, wherein the applying step is carried out by an inkjet system.

8. A method for producing cell aggregates which comprises a step of seeding sells on a substrate for producing cell aggregates provided with a spot(s) comprising a copolymer containing a recurring unit derived from a monomer represented by the following formula (I): 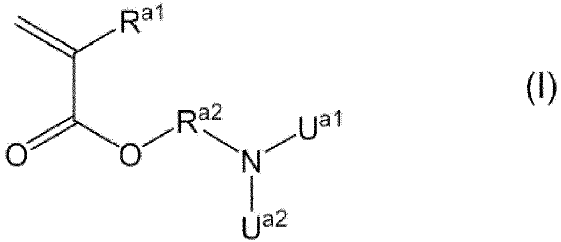 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 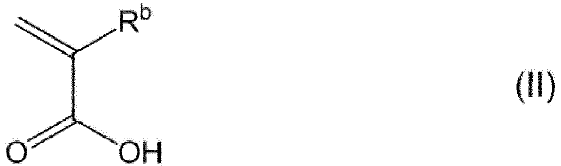 wherein
R^{b} represents a hydrogen atom or a linear or branched alkyl group having 1 to 5 carbon atoms,
on the substrate having an ability to inhibit cell adhesion, and a step of culturing the cells after seeding thereof to form cell aggregates within 20 hours.

9. A method for improving a cell culturing rate at a time of producing cell aggregates which comprises using a copolymer containing a recurring unit derived from a monomer represented by the following formula (I): 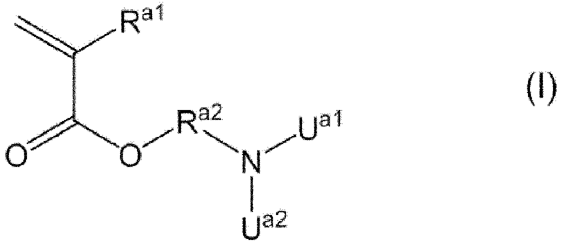 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 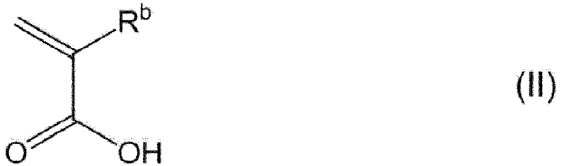 wherein
R^{b} represents a hydrogen atom or a linear or branched alkyl group having 1 to 5 carbon atoms,
as a base coat agent for cell culture on a substrate having an ability to inhibit cell adhesion, which is carried out by lowering a molar ratio of the recurring unit derived from the formula (I) to the whole copolymer.

10. A method for controlling a cell culturing rate at a time of producing cell aggregates which comprises using a copolymer containing a recurring unit derived from a monomer represented by a recurring unit derived from a monomer represented by the following formula (I): 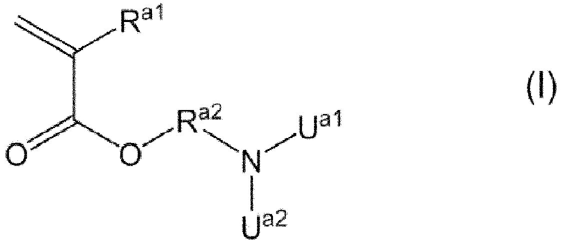 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 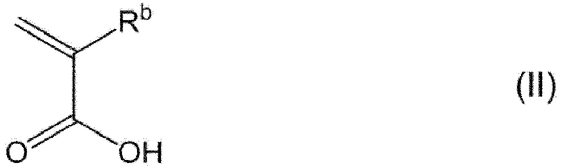 wherein
R^{b} represents a hydrogen atom or a linear or branched alkyl group having 1 to 5 carbon atoms,
as a base coat agent for cell culture on a substrate having an ability to inhibit cell adhesion, which is carried out by changing a monomer ratio of the formula (I) and the formula (II) in the copolymer.

11. A base coat agent for a cell culture container for promoting formation of cell aggregates, which comprises a copolymer containing a recurring unit derived from a monomer represented by the following formula (I): 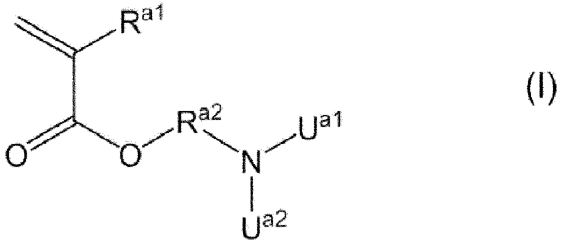 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 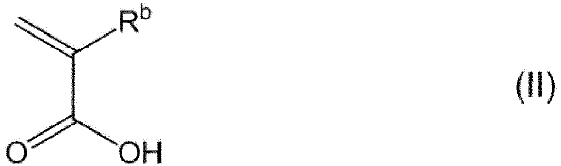 wherein
R^{b} represents a hydrogen atom or a linear or branched alkyl group having 1 to 5 carbon atoms.

12. The base coat agent according to claim 11, wherein a molar ratio of the recurring unit derived from the monomer represented by the formula (I) to a total of the recurring unit derived from the monomer represented by the formula (I) and the recurring unit derived from the monomer represented by the formula (II) is 99 mol% to 51 mol%.

13. The base coat agent according to claim 11 or 12, wherein the copolymer further contains a structural unit derived from a monomer represented by the following formula (III): 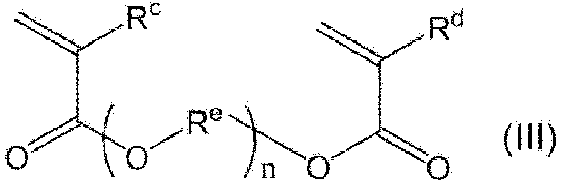 wherein
R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50.

14. The base coat agent according to any one of claims 11 to 13, wherein promotion of formation of cell aggregates is that a completion time of forming cell aggregates after seeding cells is within 20 hours.
